(19) Europäisches Patentamt
European Patent Office
Office européen des brevets

(11) Veröffentlichungsnummer: **0 249 033**
**A1**

(12) # EUROPÄISCHE PATENTANMELDUNG

(21) Anmeldenummer: **87106682.5**

(22) Anmeldetag: **08.05.87**

(51) Int. Cl.³: **C 07 D 231/28**
C 07 D 401/04, A 01 N 43/56
//C07D231/22, C07C109/04,
C07D231/18, C07D231/44,
C07D231/38

(30) Priorität: **24.05.86 DE 3617554**

(43) Veröffentlichungstag der Anmeldung:
**16.12.87 Patentblatt 87/51**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gehring, Reinhold, Dr.**
**Dasnoeckel 49**
**D-5600 Wuppertal 11(DE)**

(72) Erfinder: **Wroblowsky, Heinz-Jürgen, Dr.**
**Gladbacher Strasse 34**
**D-4018 Langenfeld(DE)**

(72) Erfinder: **Stetter, Jörg, Dr.**
**Gellertweg 4**
**D-5600 Wuppertal 1(DE)**

(72) Erfinder: **Jensen-Korte, Uta, Dr.**
**Geibelstrasse 9**
**D-4000 Düsseldorf 1(DE)**

(72) Erfinder: **Schallner, Otto, Dr.**
**Noldeweg 22**
**D-4019 Monheim(DE)**

(72) Erfinder: **Behrenz, Wolfgang, Dr.**
**Untergruendemich 14**
**D-5063 Overath(DE)**

(72) Erfinder: **Becker, Benedikt, Dr.**
**Metzkausener Strasse 14**
**D-4020 Mettmann(DE)**

(72) Erfinder: **Homeyer, Bernhard, Dr.**
**Obere Strasse 28**
**D-5090 Leverkusen 3(DE)**

(54) **5-Oxy(thio)-pyrazol-Derivate.**

(57) Es werden neue 5-Oxy(thio)-pyrazol-Derivative der allgemeinen Formel (I)

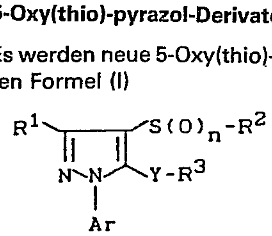

(I)

bereitgestellt,
in welcher

$R^1$ für Wasserstoff, Alkyl oder Halogenalkyl steht,

$R^2$ für gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls substituiertes Aralkyl oder für gegebenenfalls substituiertes Aryl steht,

$R^3$ für gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls substituiertes Aralkyl oder für gegebenenfalls substituiertes Aryl steht,

Ar für jeweils gegebenenfalls substituiertes Phenyl oder Pyridyl steht,

Y für O, S, SO oder $SO_2$ steht und

n für eine Zahl 0, 1 oder 2 steht

Die neuen Verbindungen (I) können nach verschiedenen im Anmeldungstext beschriebenen Methoden hergestellt werden und besitzen stark ausgeprägte insektizide, akarizide und nematizide Wirksamkeit.



Croydon Printing Company Ltd.

0249033

BAYER AKTIENGESELLSCHAFT
Konzernverwaltung RP
Patentabteilung

5090 Leverkusen, Bayerwerk

Er/bo/c
(Ib)

5-Oxy(thio)-pyrazol-Derivate

Die Erfindung betrifft neue 5-Oxy(thio)-pyrazol-Derivate, mehrere Verfahren zu ihrer Herstellung sowie ihre Verwendung als Schädlingsbekämpfungsmittel.

Es ist bereits bekannt, daß Pyrazolderivate, wie beispielsweise das 1-Cyclohexyl-5-[N,N-(dimethyl)-carbamoyloxy]-3-methylthiomethyl-pyrazol, das 1-Cyclohexyl-5-[N,N-(dimethyl)-carbamoyloxy]-3-methylsulfinylmethyl-pyrazol oder das 1-Cyclohexyl-5-[N,N-(dimethyl)-carbamoyloxy]-3-methylsulfonylmethyl-pyrazol insektizide Eigenschaften besitzen (vgl. z.B. DE-OS 28 39 270).

Die Wirkungshöhe bzw. die Wirkungsdauer dieser Verbindungen sind jedoch, insbesondere bei bestimmten lnsekten oder bei niedrigen Anwendungskonzentrationen nicht immer völlig zufriedenstellend.

Es wurden neue 5-Oxy(thio)-pyrazol-Derivate der allgemeinen Formel (I)

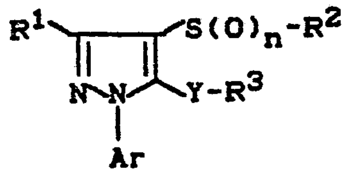
(I)

in welcher

$R^1$ für Wasserstoff, Alkyl oder Halogenalkyl steht,

$R^2$ für gegebenenfalls substituiertes Alkyl, gegebenenfalls substituiertes Alkenyl, gegebenenfalls substituiertes Alkinyl, gegebenenfalls substituiertes Cycloalkyl,gegebenenfalls substituiertes Cycloalkylalkyl, gegebenenfalls substituiertes Aralkyl oder für gegebenenfalls substituiertes Aryl steht,

$R^3$ für gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl,gegebenenfalls substituiertes Aralkyl oder für gegebenenfalls substituiertes Aryl steht,

Ar für jeweils gegebenenfalls substituiertes Phenyl oder Pyridyl steht,

Y für O, S, SO oder $SO_2$ steht und

n für eine Zahl 0, 1 oder 2 steht,

gefunden.

Weiterhin wurde gefunden, daß man die neuen 5-Oxy(thio)-pyrazol-Derivate der allgemeinen Formel (I)

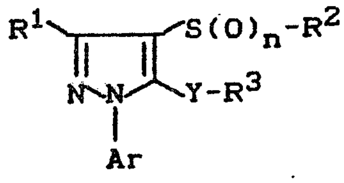
(I)

in welcher

$R^1$ für Wasserstoff, Alkyl oder Halogenalkyl steht,

$R^2$ für gegebenenfalls substituiertes Alkyl, gegebenenfalls substituiertes Alkenyl, gegebenenfalls substituiertes Alkinyl, gegebenenfalls substituiertes Cycloalkyl,gegebenenfalls substituiertes Cycloalkylalkyl, gegebenenfalls substituiertes Aralkyl oder für gegebenenfalls substituiertes Aryl steht,

$R^3$ für gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl,gegebenenfalls substituiertes Aralkyl oder für gegebenenfalls substituiertes Aryl steht,

Ar für jeweils gegebenenfalls substituiertes Phenyl oder Pyridyl steht,

Y für O, S, SO oder $SO_2$ steht und

n für eine Zahl 0, 1 oder 2 steht,

mit Hilfe eines der im folgenden beschriebenen Herstellungsverfahren erhält.

a.) Man erhält 5-Oxy(thio)-pyrazol-Derivate der Formel (Ia)

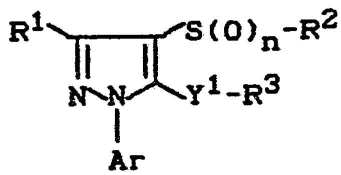

(Ia)

in welcher

$R^1$, $R^2$ ,$R^3$ ,Ar und der Index n die oben angegebene Bedeutung haben und

$Y^1$ für Sauerstoff oder Schwefel steht,

wenn man Pyrazolin-one(thione) der tautomeren Formeln (II)

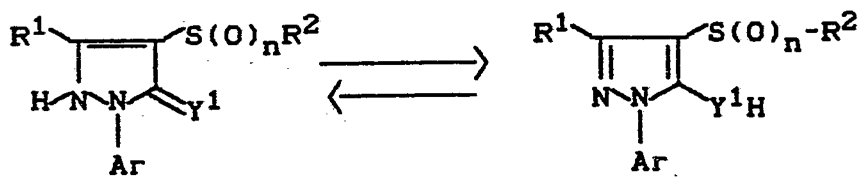

(II)

in welcher

$R^1$, $R^2$, Ar, $Y^1$ und der Index n die oben angegebene Bedeutung haben,

mit Alkylierungsmitteln der Formel (III)

$$A - R^3 \quad \text{(III)}$$

in welcher

$R^3$ die oben angegebene Bedeutung hat und

A für eine elektronenziehende Abgangsgruppe steht,

in Gegenwart eines Verdünnungsmittels und gegebenenfalls in Gegenwart eines Säurebindemittels umsetzt, wobei je nach Reaktionsbedingungen auch die entsprechenden zu (Ia) tautomeren N-alkylierten-Derivate der Formel (IV)

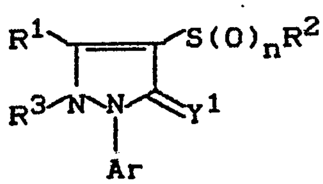

(IV)

in welcher

$R^1$, $R^2$, $R^3$, Ar, $Y^1$ und der Index n die oben angegebene Bedeutung haben

erhalten werden können.

b) Man erhält 5-Oxy(thio)-pyrazol-Derivate der Formel (Ib)

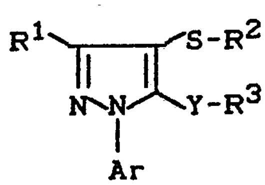

(Ib)

in welcher

$R^1$, $R^2$, $R^3$, Y und Ar die oben angegebene Bedeutung haben,

(α) wenn man Pyrazol-Derivate der Formel (V)

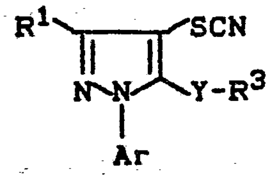

(V)

in welcher

$R^1$, $R^3$, Y und Ar die oben angegebene Bedeutung haben,

oder

(β) Bis-pyrazol-Derivate der Formel (VI)

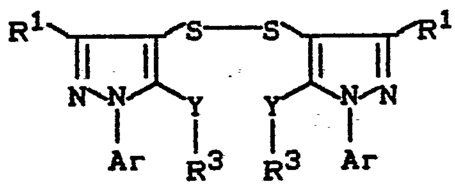

(VI)

$R^1$, $R^3$, Y und Ar die oben angegebene Bedeutung haben,

mit Alkylierungsmitteln der Formel (III a)

$$A - R^2 \qquad \text{(III a)}$$

in welcher

$R^2$ und A die oben angegebene Bedeutung haben,

gegebenenfalls in Gegenwart eines Verdünnungsmittels sowie in Gegenwart eines geeigneten Reduktionsmittels und in Gegenwart einer Base umsetzt.

(c) Man erhält 5-Oxy(thio)-pyrazol-Derivate der Formel (I), wenn man Halogen-pyrazol-Derivate der Formel (VII)

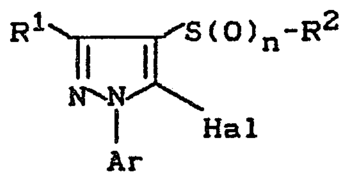

(VII)

in welcher

$R^1$, $R^2$, Ar und der Index n die oben angegebene Bedeutung hat und

Hal für Fluor, Chlor oder Brom steht,

mit Verbindungen der Formel (VIII)

$$H - Y - R^3 \qquad (VIII)$$

in welcher

$R^3$ und Y die oben angegebene Bedeutung haben,

in Gegenwart eines Verdünnungsmittels und in Gegenwart eines Säurebindemittels umsetzt.

d) Man erhält 5-Thio-pyrazol-Derivate der Formel (Ic)

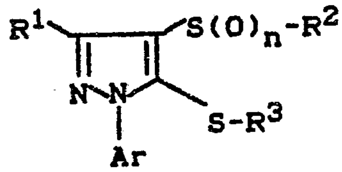

(I c)

in welcher

$R^1$, $R^2$, $R^3$, Ar und der Index n die oben angegebene Bedeutung haben,

wenn man Halogen-pyrazol-Derivate der Formel (VII)

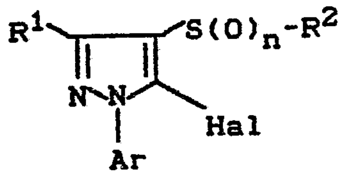

(VII)

in welcher

$R^1$, $R^2$, Ar und der Index n die oben angegebene Bedeutung haben und

Hal für Fluor, Chlor oder Brom steht,

mit Sulfenylhalogeniden der Formel (IX)

$$Hal^1 - S - R^3 \qquad (IX)$$

in welcher

$R^3$ die oben angegebene Bedeutung hat und

$Hal^1$ für Chlor oder Brom steht,

in Gegenwart einer Lithium-organischen Verbindung und in Gegenwart eines Verdünnungsmittels umsetzt.

(e) Man erhält am Schwefel in 4- oder/und 5-Stellung oxidierte 5-Oxy(thio)-pyrazol-Derivate der Formel (I), wenn man Mercapto-Derivate der Formel (I d)

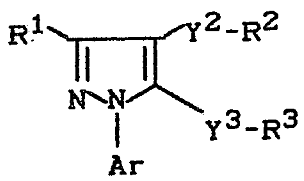

(I d)

in welcher

$R^1$, $R^2$, $R^3$ und Ar die oben angegebene Bedeutung haben und

$Y^2$ für Schwefel und

$Y^3$ für die Bedeutung von Y steht,

oder

$Y^2$ für die Gruppierungen SO oder $SO_2$ steht und

$Y^3$ für Schwefel steht,

nach bekannten Methoden in üblicher Weise oxidiert.

Schließlich wurde gefunden, daß die neuen 5-Oxy(thio)-pyrazol-Derivate der allgemeinen Formel (I) gute pestizide, insbesondere insektizide, akarizide und nematizide Eigenschaften besitzen.

Überraschenderweise zeigen die erfindungsgemäßen 5-Oxy-(thio)-pyrazol-Derivate der allgemeinen Formel (I) eine erheblich bessere insektizide bzw. akarizide bzw. nematizide Wirksamkeit als die aus dem Stand der Technik bekannten Pyrazolderivate 1-Cyclohexyl-5-[N,N-(dimethyl)-carbamoyloxy]-3-methylthiomethylpyrazol, 1-Cyclohexyl-5-[N,N-(dimethyl)-carbamoyloxy]-3-methylsulfinylmethyl-pyrazol oder 1-Cyclohexyl-5-[N,N-(dimethyl)-carbamoyloxy]-3-methylsulfonylmethyl-pyrazol, welche chemisch und wirkungsmäßig naheliegende Verbindungen sind.

Die erfindungsgemäßen 5-Oxy(thio)-pyrazol-Derivate sind durch die Formel (I) allgemein definiert. Bevorzugt sind diejenigen Verbindungen der Formel (I), bei welchen,

$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^2$ für jeweils gegebenenfalls substituiertes, geradkettiges oder verzweigtes Alkyl, Alkenyl und Alkinyl mit jeweils bis zu 6 Kohlenstoffatomen steht, wobei als Substituenten jeweils genannt seien: Halogen; Alkoxy, Alkylthio, Alkylsulfinyl und Alkylsulfonyl mit jeweils 1 bis 4 Kohlenstoffatomen, Cyano und Alkoxycarbonyl mit 1 bis 4 Kohlenstoffatomen im Alkylteil; ferner für jeweils gegebenenfalls einfach oder mehrfach, gleich oder verschieden substituiertes Cycloalkyl und Cycloalkylalkyl mit 3 bis 7 Kohlenstoffatomen im Cycloalkylteil und 1 bis 2 Kohlenstoffatomen im Alkylteil steht, wobei als Substituenten im Cycloalkylteil jeweils genannt seien: Halogen, Alkyl, Alkoxy und Alkylthio mit jeweils 1 bis 4 Kohlenstoffatomen; oder für jeweils gegebenenfalls einfach oder mehrfach, gleich oder verschieden substituiertes Phenyl und Phenylalkyl mit 1 bis 4 Kohlenstoffatomen im geradkettigen oder verzweigten Alkylteil steht, wobei als Phenylsubstituenten jeweils infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

$R^3$ für jeweils gegebenenfalls substituiertes, geradkettiges oder verzweigtes Alkyl, Alkenyl und Alkinyl mit jeweils bis zu 6 Kohlenstoffatomen steht, wobei als Substituenten jeweils genannt seien: Halogen; Alkoxy, Alkylthio, Alkylsulfinyl und Alkylsulfonyl mit jeweils 1 bis 4 Kohlenstoffatomen, Cyano und Alkoxycarbonyl mit 1 bis 4 Kohlenstoffatomen im Alkylteil;
ferner für jeweils gegebenenfalls einfach oder mehrfach, gleich oder verschieden substituiertes Cycloalkyl und Cycloalkylalkyl mit 3 bis 7 Kohlenstoffatomen im Cycloalkylteil und 1 bis 2 Kohlenstoffatomen im Alkylteil steht, wobei als Substituenten im Cycloalkylteil jeweils genannt seien: Halogen, Alkyl, Alkoxy und Alkylthio mit jeweils 1 bis 4 Kohlenstoffatomen;
oder für jeweils gegebenenfalls einfach oder mehrfach, gleich oder verschieden substituiertes Phenyl und Phenylalkyl mit 1 bis 4 Kohlenstoffatomen im geradkettigen oder verzweigten Alkylteil steht, wobei als Phenylsubstituenten jeweils infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten in Frage kommen:
Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_mR^4$

wobei

$R^4$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht;

Y für O, S, SO oder $SO_2$ steht und

n für eine Zahl 0, 1 oder 2 steht.

Besonders bevorzugt sind diejenigen Verbindungen der Formel (I), bei welchen

$R^1$ für Wasserstoff, Methyl, Ethyl, n- oder i-Propyl und Trifluormethyl steht;

$R^2$ für Methyl, Ethyl; n- oder i-Propyl; n-, i-, s- oder t-Butyl; n- oder i-Pentyl; n- oder i-Hexyl, Allyl, Propenyl, Butenyl, Propargyl oder Butinyl steht, die jeweils einfach oder mehrfach, gleich oder verschieden substituiert sein können durch Fluor, Chlor, Methoxy, Ethoxy, Methylthio, Methylsulfinyl, Methylsulfonyl, Cyano, Methoxycarbonyl oder Ethoxycarbonyl, wobei insbesondere genannt seien: Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Chlorpropenyl, Dichlorpropenyl und Chlorbutenyl;
ferner für Cyclopropyl, Cyclopentyl, Cyclohexyl, Cyclopropylmethyl, Cyclopentylmethyl oder Cyclohexylmethyl steht, die jeweils einfach oder zweifach, gleich oder verschieden durch Fluor, Chlor oder Methyl substituiert sein können;
oder für jeweils gegebenenfalls ein- bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl;

$R^3$ für Methyl, Ethyl; n- oder i-Propyl; n-, i-, s- oder t-Butyl; n- oder i-Pentyl; n- oder i-Hexyl, Allyl, Propenyl, Butenyl, Propargyl oder Butinyl steht, die jeweils einfach oder mehrfach, gleich oder verschieden substituiert sein können durch Fluor, Chlor, Methoxy, Ethoxy, Methylthio, Methylsulfinyl, Methylsulfonyl, Cyano, Methoxycarbonyl oder Ethoxycarbonyl, wobei insbesondere genannt seien: Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Chlorpropenyl, Dichlorpropenyl und Chlorbutenyl; Methoxymethyl, Methoxyethyl, Methylthiomethyl, Methylthioethyl,

Cyanomethyl, Cyanoethyl, Methoxycarbonylmethyl und Methoxycarbonylethyl; ferner für Cyclopropyl, Cyclopentyl, Cyclohexyl, Cyclopropylmethyl, Cyclopentylmethyl oder Cyclohexylmethyl steht, die jeweils einfach oder zweifach, gleich oder verschieden durch Fluor, Chlor oder Methyl substituiert sein können; oder für jeweils gegebenenfalls ein- bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl;

Ar für jeweils gegebenenfalls ein- bis fünffach, gleich oder verschieden substituiertes Phenyl, 2-Pyridyl, 3-Pyridyl oder 4-Pyridyl steht, wobei als Substituenten in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_mR^4$,

wobei

$R^4$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und

m für eine Zahl 0, 1 oder 2 steht;

Y für O, S, SO oder $SO_2$ steht und

n für eine Zahl 0, 1 oder 2 steht.

Verwendet man beispielsweise 1-(2,6-Dichlor-4-trifluormethyl-phenyl)-5-hydroxy-4-trifluormethylsulfenyl-pyrazol und Methyliodid als Ausgangsstoffe, so läßt sich der Reaktionsablauf des erfindungsgemäßen Verfahrens (a) durch das folgende Formelschema darstellen:

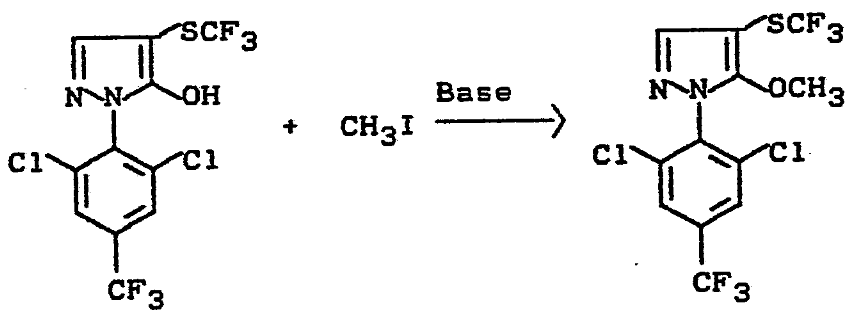

Verwendet man beispielsweise 1-(2,6-Dichlor-4-trifluormethyl-phenyl)-5-ethoxy-3-methyl-4-thiocyano-pyrazol und Methyliodid als Ausgangsstoffe und Natriumborhydrid als Reduktionsmittel, so läßt sich der Reaktionsablauf des Herstellungsverfahrens (b-α) durch das folgende Formelschema darstellen:

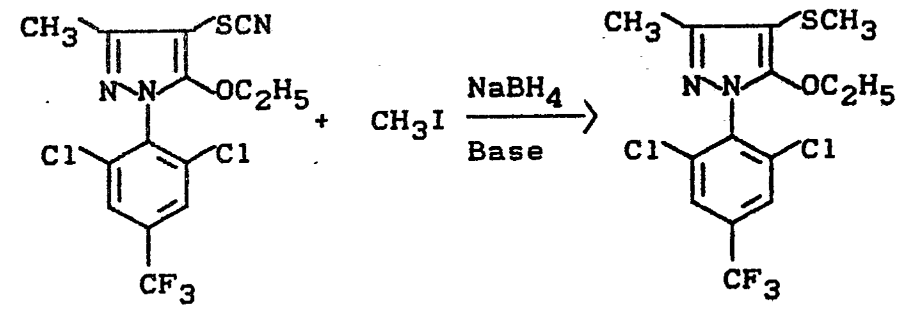


Verwendet man beispielsweise [1-(2,4-Dichlorphenyl)-5-methoxy-3-methyl-pyrazol-4-yl]-disulfid und 4-Chlorbenzylbromid als Ausgangsstoffe sowie Natriumdithionit als Reduktionsmittel, so läßt sich der Reaktionsablauf des Herstellungsverfahrens (b-β) durch das folgende Formelschema darstellen:

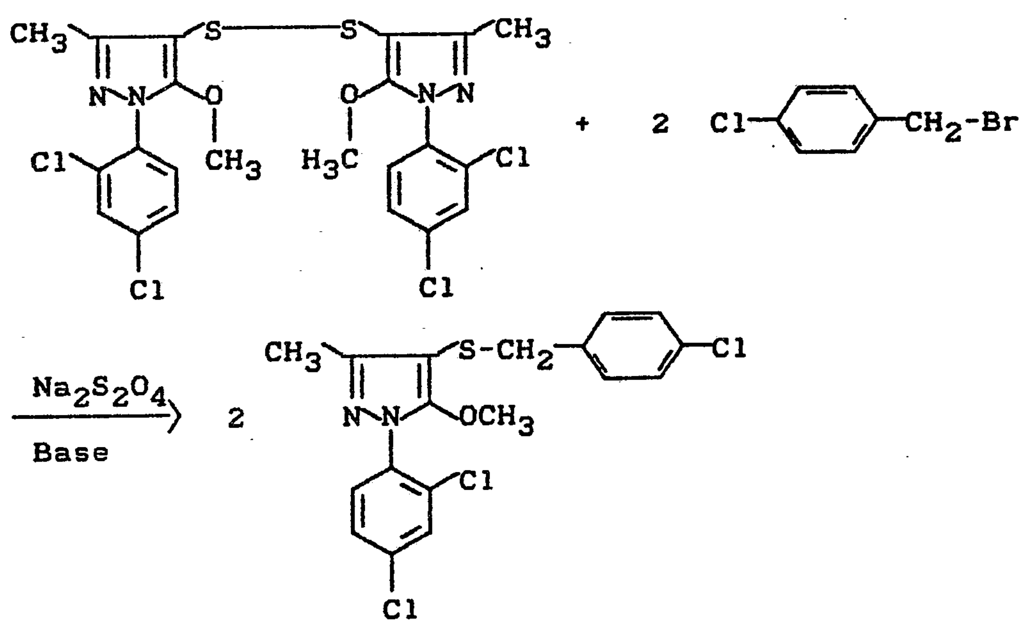

Verwendet man beispielsweise 5-Chlor-1-(2,6-dichlor-4-trifluormethyl-phenyl)-4-trifluormethylsulfonyl-pyrazol und Methylmercaptan als Ausgangsstoffe, so läßt sich der Reaktionsablauf des Herstellungsverfahrens (c) durch das folgende Formelschema darstellen:

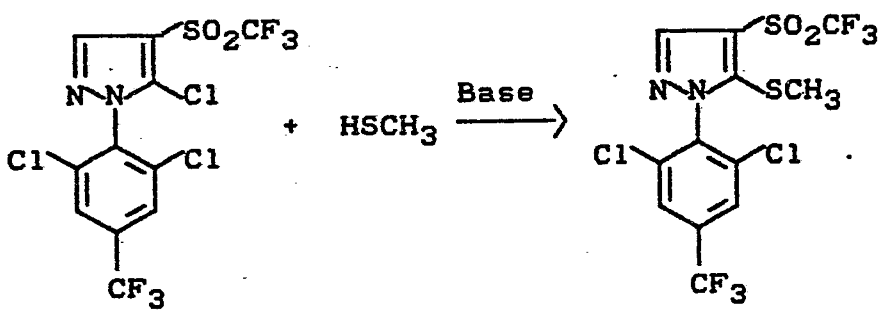


Verwendet man beispielsweise 5-Brom-1-(2,6-dichlor-4-trifluormethyl-phenyl)-3-methyl-4-trifluormethylsulfenyl-pyrazol und Dichlorfluormethansulfenylchlorid als Ausgangsstoffe und Butyl-Lithium als Lithium-organische Verbindung, so läßt sich der Reaktionsablauf des Herstellungsverfahrens (d) durch das folgende Formelschema darstellen:

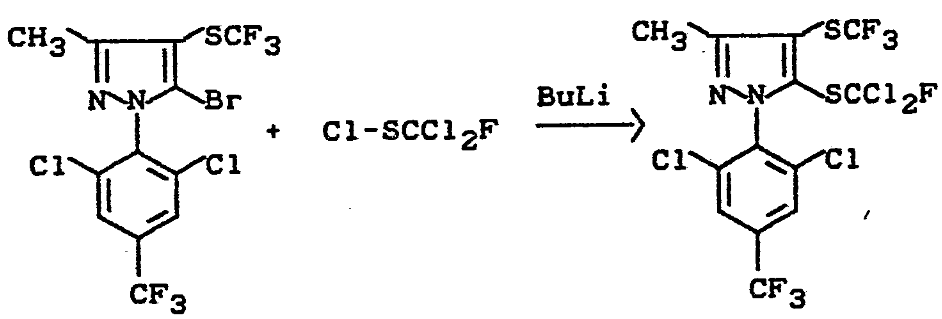


Verwendet man beispielsweise 1-(2,6-Dichlor-4-trifluormethyl-phenyl)-4-dichlorfluormethylsulfenyl-5-methoxy-pyrazol und m-Chlorperbenzoesäure als Ausgangsstoffe, so läßt

sich der Reaktionsablauf des Herstellungsverfahrens (e) durch das folgende Formelschema darstellen:

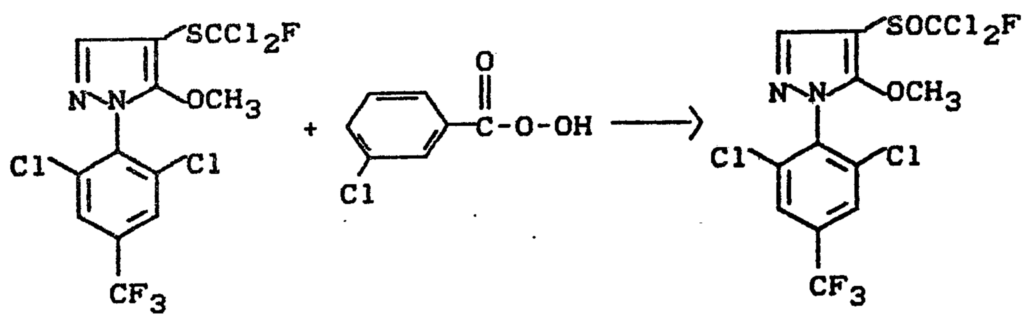

Die zur Durchführung des erfindungsgemäßen Verfahrens (a) als Ausgangsstoffe benötigten Pyrazolinone(thione) sind durch die Formel (II) allgemein definiert. In dieser Formel stehen $R^1$, $R^2$, Ar und der Index n vorzugsweise für diejenigen Bedeutungen, die bereits im Zusammenhang mit der Beschreibung der erfindungsgemäßen Stoffe der Formel (I) als bevorzugt für diese Reste bzw. diesen Index genannt wurden. $Y^1$ steht vorzugsweise für Sauerstoff oder Schwefel.

Die Pyrazolin-one(thione) der Formel (II) sind noch nicht bekannt. Sie können jedoch nach bekannten Verfahren erhalten werden, indem man z.B.

1. Pyrazolin-one der tautomeren Formeln (II a)

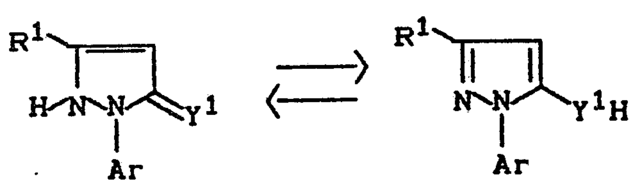

(II a)

in welcher

$R^1$, Ar und $Y^1$ die oben angegebene Bedeutung haben,

mit Sulfenylchloriden der Formel (IX a)

$$Hal^1 - S - R^2 \qquad \text{(IX a)}$$

in welcher

$R^2$ und $Hal^1$ die oben angegebene Bedeutung haben,

in Gegenwart eines inerten organischen Lösungsmittels, wie beispielsweise Methylenchlorid, und in Gegenwart einer Base, wie beispielsweise Pyridin, bei Temperaturen zwischen 10 und 60°C umsetzt (vgl. auch die Herstellungsbeispiele); oder

2. Ketocarbonsäure-Derivate der Formel (X)

$$R^1-\overset{\overset{O}{\|}}{C}-\overset{\overset{SR^2}{|}}{CH}-COOR^4 \qquad \text{(X)}$$

in welcher

$R^1$ und $R^2$ die oben angegebene Bedeutung haben und

$R^4$ für Alkyl mit 1 bis 4 Kohlenstoffatomen, wie vorzugsweise Methyl oder Ethyl steht,

mit Hydrazinen der Formel (XI)

$H_2N - NH - Ar$ (XI)

in welcher

Ar die oben angegebene Bedeutung hat,

in Gegenwart eines inerten organischen Lösungsmittels, wie beispielsweise Toluol, und in Gegenwart eines Katalysators, wie beispielsweise p-Toluolsulfonsäure, bei Temperaturen zwischen 0 und 100° C umsetzt; und gegebenenfalls

3. die nach den Varianten 1 und 2 erhaltenen Mercapto-Derivate der Formel (II b)

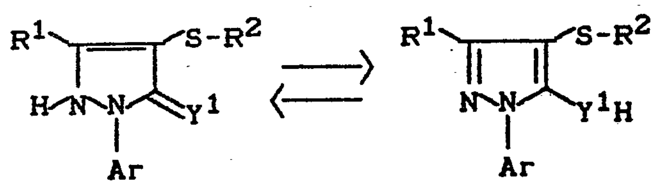

(II b)

in welcher

$R^1$, $R^2$, Ar und $Y^1$ die oben angegebene Bedeutung haben,

nach bekannten Methoden gemäß Verfahren (e) am Schwefel der Sulfenylgruppe in 4-Position des Pyrazolrings oxidiert.

Die Pyrazolin-one der Formel (II a) können in bekannter Art und Weise erhalten werden (vgl. hierzu auch die

Deutsche Patentanmeldung P 35 01 323 vom 17.01.1985), indem man

- Alkoxymethylmalonester der Formel (XII)

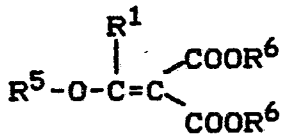 (XII)

in welcher

$R^1$ die oben angegebene Bedeutung hat und

$R^5$ und $R^6$ unabhängig voneinander jeweils für Alkyl, insbesondere für Methyl oder Ethyl stehen,

mit Hydrazinen der Formel (XI)

$$H_2N - NH - Ar \quad (XI)$$

in welcher

Ar die oben angegebene Bedeutung hat,

zunächst in einer ersten Stufe gegebenenfalls in Gegenwart eines Verdünnungsmittels, wie beispielsweise Methanol oder Ethanol, bei Temperaturen zwischen +10° C und +80° C umsetzt; wobei

das intermediär auftretende Zwischenprodukt der Formel (XI a)

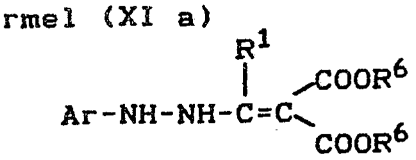

(XI a)

in welcher

Ar, $R^1$ und $R^6$ die oben angegebene Bedeutung haben,

gegebenenfalls isoliert und separat cyclisiert werden kann;

und die so erhaltenen Pyrazolcarbonsäureester der Formel (XIII)

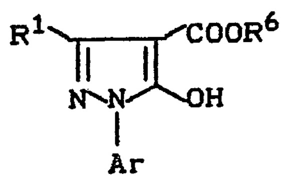

(XIII)

in welcher

Ar, $R^1$ und $R^6$ die oben angegebene Bedeutung haben,

in einer 2. Stufe gegebenenfalls in Gegenwart eines Verdünnungsmittels, wie beispielsweise Methanol, und gegebenenfalls in Gegenwart einer Base, wie beispielsweise Natriumhydroxid, bei Temperaturen zwischen 30° C und 70° C decarboxyliert (vgl. auch die Herstellungsbeispiele).

Die Cyclisierung und anschließende Decarboxylierung kann gegebenenfalls auch in einer Reaktionsstufe als 'Eintopfverfahren' durchgeführt werden (vgl. z.B. Liebigs Ann. Chem. 373, 142 (1910); oder indem man

- Acylessigester der allgemeinen Formel (XIV)

$$R^1\text{-CO-CH}_2\text{-COOR}^6 \qquad \text{(XIV)}$$

in welcher

$R^1$ und $R^6$ die oben angegebene Bedeutung haben,

mit Hydrazinen der Formel (XI)

$$H_2N\text{-NH-Ar} \qquad \text{(XI)}$$

in welcher

Ar die oben angegebene Bedeutung hat,

in Gegenwart eines inerten organischen Lösungsmittels bei Temperaturen zwischen 20°C und 100°C umsetzt (vgl. hierzu A.Weissberger 'The Chemistry of Heterocyclic Compounds'; Pyrazoles, Pyrazolines, Pyrazolidines, Indazoles and Condensed rings; 1967).

Die zur Herstellung der Pyrazolin-one der Formel (II a) aufgeführten Vorprodukte Alkoxymethylmalonester der Formel

(XII), Hydrazine der Formel (XI) und Acylessigester der Formel (XIV) sind bekannt, bzw. können sie in bekannter Art und Weise erhalten werden.

Die Pyrazolin-thione der Formel (II a) können erhalten werden, indem man

- Pyrazolin-one der Formel (II-c)

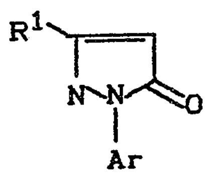

(II c)

in welcher

Ar und $R^1$ die oben angegebene Bedeutung haben,

in bekannter Art und Weise mit Schwefelreagenzien, wie beispielsweise Phosphorpentasulfid, in einem inerten organischen Lösungsmittel, wie beispielsweise Toluol, umsetzt (vgl. hierzu auch A.Weissberger 'The Chemistry of Heterocyclic Compounds'; Pyrazolones, Pyrazolidones and Derivates; 1964); oder indem man

- 5-Halogenpyrazole der Formel (XV)

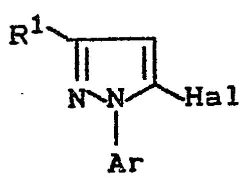

(XV)

in welcher

Ar, $R^1$ und Hal die oben angegebene Bedeutung haben,

in bekannter Art und Weise mit Kaliumhydrogensulfid umsetzt (vgl. hierzu A.Weissberger 'The Chemistry of Heterocyclic Compounds'; Pyrazolones, Pyrazolidones and Derivatives; 1964).

Die 5-Halogenpyrazole der Formel (XV) können erhalten werden, indem man z.B. Pyrazolin-one der Formel (II a) mit üblichen Halogenierungsmitteln, wie beispielsweise Phosphoroxychlorid oder Phosphoroxybromid umsetzt (vgl. hierzu Berichte <u>28</u>, 35 (1895) oder Liebigs Annalen der Chemie <u>373</u>, 129 (1910)).

Die Ketocarbonsäure-Derivate der Formel (X) können erhalten werden, indem man Acylessigester der Formel (X a)

$$R^1-\overset{\overset{\displaystyle O}{\|}}{C}-CH_2-COOR^4 \qquad (X\ a)$$

in welcher

$R^1$ und $R^2$ die oben angegebene Bedeutung haben,

mit Sulfenylhalogeniden der Formel (IX a)

$$Hal^1 - S - R^2 \qquad (IX\ a)$$

in welcher

$R^2$ und $Hal^1$ die oben angegebene Bedeutung haben,

in Gegenwart eines inerten organischen Lösungsmittels, wie beispielsweise Methylenchlorid, und in Gegenwart einer Base, wie beispielsweise Pyridin, bei Temperaturen zwischen 10 und 60°C umsetzt.

Die außerdem zur Durchführung des erfindungsgemäßen Verfahrens (a) als Ausgangsstoffe benötigten Alkylierungsmittel sind durch die Formel (III) allgemein definiert. In dieser Formel steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden. A steht vorzugsweise für Chlor, Brom, Iod, p-Toluolsulfonyloxy oder Methoxysulfonyloxy.

Die Alkylierungsmittel der Formel (III) sind allgemein bekannte Verbindungen der organischen Chemie.

Die zur Durchführung des erfindungsgemäßen Verfahrens (b-α) als Ausgangsstoffe benötigten Pyrazol-Derivate sind durch die Formel (V) allgemein definiert. In dieser Formel stehen $R^1$, $R^3$, Y und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die Pyrazol-Derivate der Formel (V) sind noch nicht bekannt. Sie können jedoch nach bekannten Verfahren erhalten werden, indem man z.B. 4-unsubstituierte Pyrazol-Derivate der Formel (XVI)

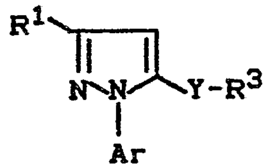

(XVI)

in welcher

$R^1$, $R^3$, Y und Ar die oben angegebene Bedeutung haben,

mit Ammoniumthiocyanat in Gegenwart von Brom und Essigsäure bei Temperaturen zwischen -20° C und +20° C umsetzt (vgl. auch die Herstellungsbeispiele).

Die 4-unsubstituierten Pyrazol-Derivate der Formel (XVI) können in bekannter Art und Weise erhalten werden, indem man Pyrazolin-one(thione) der Formel (II a) mit Alkylierungsmitteln der Formel (III) gemäß den Bedingungen des Verfahrens (a) umsetzt (vgl. auch die Herstellungsbeispiele).

Die zur Durchführung des erfindungsgemäßen Verfahrens (b-β) als Ausgangsstoffe benötigten Bis-pyrazol-Derivate sind durch die Formel (VI) allgemein definiert. In dieser Formel stehen $R^1$, $R^3$, Y und Ar vorzugsweise für diejenigen Reste, die bereits im Zusammenhang mit der Beschreibung der erfindungsgemäßen Stoffe der Formel (I) als bevorzugt für diese Substituenten genannt wurden.
Die Bis-pyrazol-Derivate der Formel (VI) sind noch nicht bekannt. Sie können jedoch nach bekannten Verfahren erhalten werden, indem man z.B. die oben beschriebenen Pyrazol-Derivate der Formel (V) mit wäßriger Salzsäure gegebenenfalls in Gegenwart eines Verdünnungsmittels, wie beispielsweise Ethanol, bei Temperaturen zwischen 20°C und 120°C umsetzt.

Die außerdem zur Durchführung der erfindungsgemäßen Verfahren (b-α) und (b-β) als Ausgangsstoffe benötigten Alkylierungsmittel sind durch die Formel (III a) allgemein definiert. In dieser Formel steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden. A steht vorzugsweise für Chlor, Brom, Iod, p-Toluolsulfonyloxy oder Methoxysulfonyloxy.

Die Alkylierungsmittel der Formel (III a) sind allgemein bekannte Verbindungen der organischen Chemie.

Die zur Durchführung der erfindungsgemäßen Verfahren (c) und (d) als Ausgangsstoffe benötigten Halogen-pyrazol-Derivate sind durch die Formel (VII) allgemein definiert. In dieser Formel stehen $R^1$, $R^2$, Ar und der Index n vorzugsweise für diejenigen Bedeutungen, die bereits im Zusammenhang mit der Beschreibung der erfindungsgemäßen Stoffe der Formel (I) als bevorzugt für diese Reste bzw. diesen Index genannt wurden. Hal steht vorzugsweise für Chlor oder Brom.

Die Halogen-pyrazol-Derivate der Formel (VII) sind noch nicht bekannt; sie sind jedoch teilweise Gegenstand einer eigenen, älteren Patentanmeldung, die noch nicht veröffentlicht ist (vgl. die Deutsche Patentanmeldung P 35 29 829 vom 21.08.1985 [LeA 23 917]); und können nach den

dort beschriebenen Verfahren erhalten werden, indem man 5-Amino-pyrazol-Derivate der Formel (XVII)

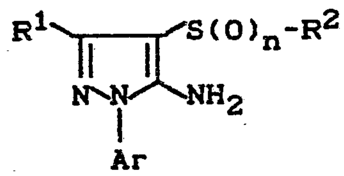

(XVII)

in welcher

$R^1$, $R^2$, Ar und der Index n die oben angegebene Bedeutung haben,

mit einem anorganisch oder organischen Nitrit, wie beispielsweise Natriumnitrit oder tert.-Butylnitrit, in Gegenwart eines Reaktionshilfsmittels, wie beispielsweise einer Halogenwasserstoffsäure oder Haloform, vorzugsweise seien Chlorwasserstoffsäure, Bromwasserstoffsäure, Chloroform und Bromoform genannt, bei Temperaturen zwischen -30° C und +60° C umsetzt.

Die 5-Amino-pyrazol-Derivate der Formel (XVII) sind bekannt (vgl. DE-OS 34 02 308 [LeA 22 853]), bzw. sind sie Gegenstand einer eigenen älteren Patentanmeldung, die noch nicht veröffentlicht ist (vgl. die Deutsche Patentanmeldung P 35 17 843 vom 17.05.1985 [LeA 23 753]), bzw. können sie nach den dort angegebenen Verfahren erhalten werden, indem man 4-Thiocyanato-5-aminopyrazole der Formel (XVIII)

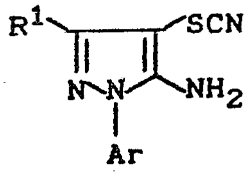

(XVIII)

in welcher

$R^1$ und Ar die oben angegebene Bedeutung haben,

oder Bis-pyrazolyl-disulfide der Formel (XIX)

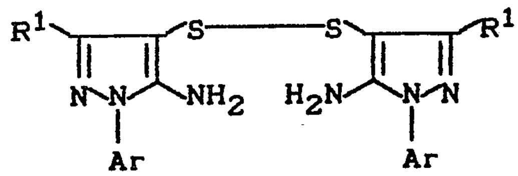

(XIX)

in welcher

$R^1$ und Ar die oben angegebene Bedeutung haben,

mit Alkylierungsmitteln der Formel (III a)

$$A - R^2 \qquad \text{(III a)}$$

in welcher

$R^2$ und A die oben angegebene Bedeutung haben,

gegebenenfalls in Gegenwart eines Verdünnungsmittels wie beispielsweise Methanol oder Ethanol sowie gegebenenfalls in Gegenwart eines Reduktionsmittels wie beispielsweise Natriumborhydrid oder Natriumdithionit und gegebenenfalls

in Gegenwart einer Base, wie beispielsweise Natriumhydroxid oder Kaliumcarbonat bei Temperaturen zwischen 20° C und 90° C umsetzt, oder wenn man

4-unsubstituierte 5-Amino-pyrazole der Formel (XX)

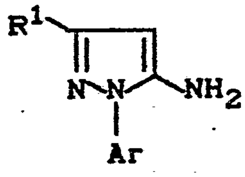

(XX)

in welcher

$R^1$ und Ar die oben angegebene Bedeutung haben,

mit Sulfenylhalogeniden der Formel (IX a)

$$Hal^1 - S - R^2 \qquad (IX\ a)$$

in welcher

$R^2$ und $Hal^1$ die oben angegebene Bedeutung haben,

gegebenenfalls in Gegenwart eines Verdünnungsmittels wie beispielsweise Dichlormethan und gegebenenfalls in Gegenwart eines Säurebindemittels wie beispielsweise Pyridin bei Temperaturen zwischen 0° C und 50° C umsetzt, und gegebenenfalls anschließend die so erhältlichen 5-Amino-pyrazole der Formel (XVII a)

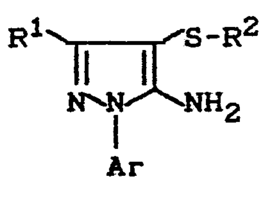

(XVII a)

in welcher

$R^1$, R und Ar die oben angegebene Bedeutung haben,

nach bekannten Methoden in üblicher Weise entsprechend Verfahren (e) oxidiert.

Die 4-Thiocyanato-5-aminopyrazole der Formel (XVIII) können beispielsweise erhalten werden, wenn man entsprechende 4-unsubstituierte 5-Aminopyrazole der Formel (XX) mit Ammoniumthiocyanat in Gegenwart von Brom und Essigsäure bei Temperaturen zwischen -20°C und +20°C umsetzt.

Die Bis-pyrazolyl-disulfide der Formel (XIX) können erhalten werden, wenn man die oben beschriebenen 4-Thiocyanato-5-aminopyrazole der allgemeinen Formel (XVIII) mit wässriger Salzsäure, gegebenenfalls in Gegenwart eines Verdünnungsmittels, wie beispielsweise Ethanol, bei Temperaturen zwischen 20°C und 120°C umsetzt.

Die 4-unsubstituierten 5-Aminopyrazole der allgemeinen Formel (XX) sind bekannt (vgl. z.B. J.Org.Chem. 36,2972-2974 (1971); J.Heterocyclic Chemistry 7, 345-349 (1970); DE-OS 34 02 308 [LeA 22 853]), bzw. sind sie Gegenstand einer eigenen älteren Patentanmeldung, die noch nicht veröffentlicht ist (vgl. die Deutsche Patentanmeldung P 35 17 843 vom 17.05.1985 [LeA 23 753]), bzw. können sie nach den dort angegebenen Verfahren erhalten werden.

Die Acylierungsmittel der allgemeinen Formel (III a) sowie die Sulfenylhalogenide der allgemeinen Formel (IX a) sind allgemein bekannte Verbindungen der organischen Chemie.

Die außerdem zur Durchführung des erfindungsgemäßen Verfahrens (c) als Ausgangsstoffe benötigten Verbindungen sind durch die Formel (VIII) allgemein definiert. In dieser Formel stehen $R^3$ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Verbindungen der Formel (VIII) sind allgemein bekannte Verbindungen der organischen Chemie.

Die außerdem zur Durchführung des erfindungsgemäßen Verfahrens (d) als Ausgangsstoffe benötigten Sulfenylhalogenide sind durch die Formel (IX) allgemein definiert. In dieser Formel steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Sulfenylhalogenide der Formel (IX) sind allgemein bekannte Verbindungen der organischen Chemie.

Die zur Durchführung des erfindungsgemäßen Verfahrens (e) als Ausgangsstoffe zu verwendenden Verbindungen sind durch die Formel (I d) allgemein definiert. Die Verbindungen der Formel (I d) sind erfindungsgemäße Stoffe.

Die erfindungsgemäße Oxidation erfolgt durch Umsetzung mit üblichen anorganischen und organischen Oxidationsmitteln. Hierzu gehören vorzugsweise organische Persäuren, wie z.B. Peressigsäure, p-Nitroperbenzoesäure, m-Chlorperbenzoesäure; anorganische Persäuren, wie z.B. Periodsäure; weiterhin Wasserstoffperoxid in Eisessig oder Methanol, Kaliumpermanganat oder Chromsäure.

Als Verdünnungsmittel zur Durchführung der Herstellungsverfahren (a) und (c) kommen inerte organische Lösungsmittel in Frage.
Hierzu gehören insbesondere aliphatische und aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Alkohole, wie Methanol, Ethanol oder Isopropanol, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.
In manchen Fällen erweist es sich als vorteilhaft, in einem wässrig-organischen Zweiphasensystem zu arbeiten.

Die Herstellungsverfahren (a) und (c) werden gegebenenfalls in Gegenwart eines Säurebindemittels durchgeführt.

Als solche kommen alle üblichen anorganischen oder organischen Basen in Frage. Hierzu gehören beispielsweise Al-

kalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei dem erfindungsgemäßen Verfahren (a) in einem größeren Bereich variiert werden. Im allgemeinen arbeitet man bei Temperaturen zwischen -20° C und +80 °C, vorzugsweise bei Temperaturen zwischen -10 °C und +40 °C.

Die Reaktionstemperaturen können bei dem erfindungsgemäßen Verfahren (c) in einem größeren Bereich variiert werden. Im allgemeinen arbeitet man bei Temperaturen zwischen -20 °C und +120 °C, vorzugsweise bei Temperaturen zwischen 0 °C und +50 °C.

Zur Durchführung des Herstellungsverfahrens (a) setzt man pro Mol Pyrazolin-on(thion) der Formel (II) im allgemeinen 1 bis 10 Mol, vorzugsweise 1 bis 5 Mol an Alkylierungsmittel der Formel (III) und gegebenenfalls 1 bis 5 Mol an Säurebindemittel ein. Die Reaktionsführung, Aufarbeitung und Isolierung der Reaktionsprodukte erfolgt nach allgemein bekannten und üblichen Verfahren.

Zur Durchführung des Herstellungsverfahrens (c) setzt man pro Mol Halogen-pyrazol-Derivat der Formel (VII) im allgemeinen 1 bis 2,5 Mol, vorzugsweise 1 bis 1,5 Mol an Verbindung der Formel (VIII) und 1 bis 1,5 Mol Säurebindemittel ein. Die Reaktionsführung, Aufarbeitung und Isolierung der Reaktionsprodukte erfolgt nach allgemein bekannten und üblichen Verfahren.

Als Verdünnungsmittel zur Durchführung der Herstellungsverfahren (b-α) und (b-β) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Alkohole, wie Methanol, Ethanol oder Propanol.

Als Reduktionsmittel zur Durchführung des Herstellungsverfahrens (b-α) verwendet man vorzugsweise komplexe Hydride wie Lithiumaluminiumhydrid, Lithiumborhydrid oder Natriumborhydrid. Besonders geeignet ist Natriumborhydrid.

Als Reduktionsmittel zur Durchführung des Herstellungsverfahrens (b-β) kommen alle üblicherweise für Disulfidspaltungen verwendbaren Reduktionsmittel in Frage. Mit besonderem Vorzug verwendet man Dithionite, wie beispielsweise Natriumdithionit.

Die Herstellungsverfahren (b-α) und (b-β) werden in Gegenwart einer geeigneten Base durchgeführt. Vorzugsweise verwendet man Alkalimetallhydroxide oder -carbonate, wie beispielsweise Natrium- oder Kaliumhydroxid oder Natrium- oder Kaliumcarbonat.

Die Reaktionstemperaturen können bei der Durchführung der Herstellungsverfahren (b-α) und (b-β) in einem größeren Bereich variiert werden. Im allgemeinen arbeitet man bei Temperaturen zwischen 0° C und +120° C, vorzugsweise bei Temperaturen zwischen +20° C und +90° C.

Zur Durchführung des Herstellungsverfahrens (b-α) setzt man pro Mol an Pyrazol-Derivat der Formel (V) im allgemeinen 1,0 bis 5,0 Mol, vorzugsweise 1,8 bis 2,5 Mol an Alkylierungsmittel der Formel (III a) und 1,0 bis 5,0 Mol, vorzugsweise 1,8 bis 2,0 Mol an Reduktionsmittel sowie 1,0 bis 5,0 Mol, vorzugsweise 1,5 bis 3,0 Mol an Base ein. Dabei setzt man zunächst das Pyrazol-Derivate der Formel (V) in dem betreffenden Verdünnungsmittel unter Verwendung einer Stickstoff-Schutzgasatmosphäre mit dem Reduktionsmittel um und setzt nach beendeter Reaktion die Base und das Alkylierungsmittel der Formel (III a) zu. Die Aufarbeitung und Isolierung der Reaktionsprodukte der Formel (I b) erfolgt nach üblichen Verfahren.

Zur Durchführung des Herstellungsverfahrens (b-β) setzt man pro Mol an Bis-pyrazol-Derivat der Formel (VI) im allgemeinen 1,0 bis 5,0 Mol, vorzugsweise 1,8 bis 2,5 Mol an Alkylierungsmittel der Formel (III a) und 1,0 bis 5,0 Mol, vorzugsweise 1,8 bis 2,0 Mol an Reduktionsmittel sowie 1,0 bis 5,0 Mol, vorzugsweise 1,5 bis 3,0 Mol an Base ein. Dabei setzt man zunächst das Bis-pyrazol-Derivat der Formel (VI) in dem betreffenden Verdünnungsmittel in Gegenwart der Base bei der entsprechenden Reaktionstemperatur mit dem Reduktionsmittel um, setzt nach einigen Stunden das Alkylierungsmittel der Formel (III a) zu und erwärmt eine weitere Stunde auf die erforderliche Reaktionstemperatur. Die Aufarbeitung und Isolierung der Reaktionsprodukte erfolgt nach üblichen Verfahren.

Als Verdünnungsmittel zur Durchführung des Herstellungsverfahrens (d) kommen inerte organische Lösungsmittel infrage. Hierzu gehören insbesondere aliphatische oder aromatische Kohlenwasserstoffe, wie beispielsweise Benzin, Benzol, Toluol, Xylol, Petrolether, Hexan, Cyclohexan oder Ether wie Diethylether, Dioxan, Tetrahydrofuran oder Ethylenglykoldimethyl- oder -diethylether.

Das Herstellungsverfahren (d) wird in Gegenwart von Lithium-organischen Verbindungen durchgeführt. Hiebei seien vorzugsweise genannnt: Butyl-Lithium, Phenyllithium und Lithiumdiisopropylamid.

Die Reaktionstemperaturen können bei der Durchführung des Herstellungsverfahrens (d) in einem größeren Bereich variiert werden. Im allgemeinen arbeitet man bei Temperaturen zwischen -80° C und +50° C, vorzugsweise bei Temperaturen zwischen -80° C und +30° C.

Zur Durchführung des Herstellungsverfahrens (d) setzt man pro Mol Halogen-pyrazol-Derivat der Formel (VII) 1 bis 1,5 Mol, vorzugsweise 1 bis 1,3 Mol an Lithium-organischer Verbindung und 1 bis 10 Mol, vorzugsweise 1 bis 2 Mol Sulfenylhalogenid der Formel (IX) ein.

Dabei setzt man in allgemein üblicher Art und Weise zunächst das 5-Halogen-1-aryl-pyrazol der Formel (VII)

unter Luft- und Feuchtigkeitsausschluß mit der Lithiumorganischen Verbindung um und gibt anschließend das Sulfenylhalogenid der Formel (IX) in die Reaktionsmischung. Die Aufarbeitung und Isolierung der Reaktionsprodukte der Formel (I c) erfolgt nach allgemein üblichen Verfahren.

Die Reaktionstemperaturen können bei der Durchführung der Oxidation gemäß Herstellungsverfahren (e) in einem größeren Bereich variiert werden. Im allgemeinen arbeitet man zwischen -50° C und 100° C, vorzugsweise zwischen -30° C und 80° C.

Bei der Durchführung der erfindungsgemäßen Oxidation gemäß Herstellungsverfahren (e) setzt man auf 1 Mol Mercapto-Derivat der Formel (I d) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Acetanhydrid bei Temperaturen zwischen -30° C bis +30° C, entstehen vorzugsweise die erfindungsgemäßen Verbindungen der Formel (I) mit SO-Gruppierung. Bei Überschuß an Oxidationsmittel und höheren Temperaturen (10 bis 80° C) entstehen vorzugsweise die erfindungsgemäßen Verbindungen der Formel (I) mit $SO_2$-Gruppierung. Die Isolierung der Oxidationsprodukte erfolgt in üblicher Weise.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäß Verwendbaren Wirkstoffe der Formel (I) zeichnen sich durch eine hohe insektizide, akarizide und nematizide Wirksamkeit aus. Sie lassen sich insbesondere gegen pflanzenschädigende Insekten, wie beispielsweise gegen die Larven der Meerrettichblattkäfer (Phaedon cochleariae) einsetzen. Sie eignen sich daneben hervorragend zur Bekämpfung von Bodeninsekten und Nematoden und lassen sich beispielsweise zur Bekämpfung von Phorbia antiqua-Maden einsetzen.

Außerdem besitzen die erfindungsgemäß verwendbaren Wirkstoffe der Formel (I) eine hohe Wirkung gegen Hygiene- und Vorratsschädlinge und lassen sich beispielsweise zur Bekämpfung der Stubenfliege (Musca domestica) oder zur Bekämpfung des gemeinen Kornkäfers (Sitophilus granarius) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

## Herstellungsbeispiele

### Beispiel 1

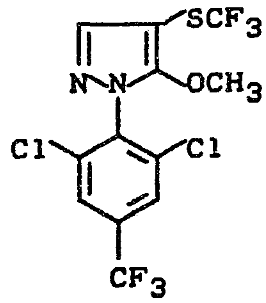

(I-1)

(Verfahren a)

Zu einer Mischung von 7,4g (0,02 Mol) 1-(2,6-Dichlor-4-trifluormethyl-phenyl)-5-hydroxy-4-trifluormethylsulfenyl-pyrazol und 1,1g gepulvertem Kaliumhydroxid in 40ml heißem Dimethylsulfoxid gibt man 9,6g (0,066 Mol) Iodmethan. Man läßt das Reaktionsgemisch über Nacht bei Raumtemperatur rühren. Danach gibt man auf Wasser, extrahiert mit Methylenchlorid, trocknet und engt ein. Nach säulenchromatographischer Trennung (Methylenchlorid/Aceton = 9 : 1) erhält man 1,3g (16% der Theorie) 1-(2,6-Dichlor-4-trifluormethyl-phenyl)-5-methoxy-4-trifluormethylsulfenyl-pyrazol vom Schmelzpunkt 79-84° C.

### Herstellung der Vorprodukte

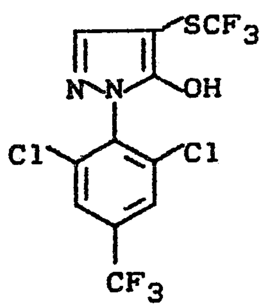

(II-1)

17,8g (0,06 Mol) 1-(2,6-Dichlor-4-trifluormethylphenyl)-5-hydroxy-pyrazol werden in 200 ml Methylenchlorid suspendiert und auf 0°C abgekühlt. Diese Suspension wird zunächst mit 5,4g (0,066 Mol) Pyridin versetzt und anschließend werden 8,7g (0,063 Mol) Trifluormethansulfenylchlorid eingeleitet. Man läßt das Reaktionsgemisch über Nacht bei Raumtemperatur rühren. Zur Aufarbeitung wird das Lösungsmittel abgezogen, und der schmierige Rückstand durch Anreiben mit Wasser zur Kristallisation gebracht. Das Rohprodukt wird mit Ether extrahiert (Soxhlet) und das Lösungsmittel abdestilliert.

Man erhält 13,3g (60% der Theorie) 1-(2,6-Dichlor-4-trifluormethyl-phenyl)-5-hydroxy-4-trifluormethyl-sulfenyl-pyrazol vom Schmelzpunkt 144-152°C.

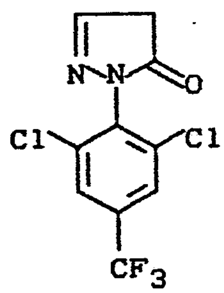

(IIa-1)

100g (0,24 Mol) N-[2,2-Bis(ethoxycarbonyl)-vinyl]-N'-(2,6-dichlor-4-trifluormethyl-phenyl)-hydrazin und 200g (5 Mol) Natriumhydroxid werden in 1400 ml Wasser 12 Stunden unter Rückfluß erhitzt. Die erkaltete Reaktionsmischung wird unter heftigem Rühren mit konzentrierter Salzsäure angesäuert ($CO_2$-Entwicklung) der ausgefallene Feststoff wird abgesaugt, an der Luft getrocknet und aus Ethanol/Wasser umkristallisiert.

Man erhält 41g (56% der Theorie) 1-(2,6-Dichlor-4-trifluormethyl-phenyl)-4H-$\Delta^2$-pyrazolin-5-on vom Schmelzpunkt 220°C.

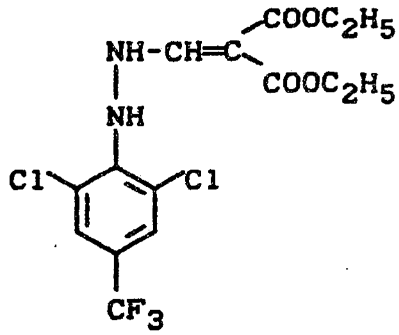

(XIa-1)

24,5g (0,1 Mol) 2,6-Dichlor-4-trifluormethyl-phenylhydrazin und 21,6g (0,1 Mol) Ethoxymethylenmalonsäurediethylester werden 24 Stunden bei Raumtemperatur gerührt, danach wird eingeengt und der kristalline Rückstand mit Petrolether verrührt. Der Rückstand wird abgesaugt und an der Luft getrocknet.
Man erhält 33g (79,5% der Theorie) an N-[2,2-Bis-(ethoxycarbonyl)-vinyl]-N'-(2,6-dichlor-4-trifluormethyl-phenyl)-hydrazin vom Schmelzpunkt 86°C.

Beispiel 2

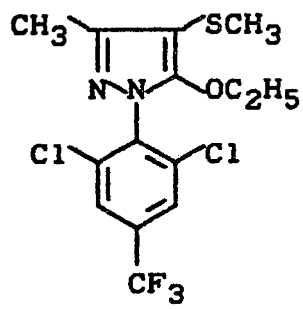

(I-2)

(Verfahren b-α)
8g (0,02 Mol) 1-(2,6-Dichlor-4-trifluormethyl-phenyl)-5-ethoxy-3-methyl-4-thiocyano-pyrazol in 100 ml absolutem Methanol werden bei Raumtemperatur in einer Stickstoffatmosphäre portionsweise mit 1,4g (0,037 Mol) Natriumborhydrid versetzt. Nach beendeter Zugabe rührt man 45 Minuten bei Raumtemperatur und gibt dann 2,2g (0,04 Mol) Kaliumhydroxid und anschließend tropfenweise 7g (0,04 Mol) Methyliodid zu. Nach beendeter Zugabe rührt man 1 Stunde bei Raumtemperatur nach, engt im Vakuum ein, nimmt den Rückstand in Wasser auf, extrahiert mit Chloroform, trocknet über Natriumsulfat und engt im Vakuum ein.
Man erhält 3,4g (44% der Theorie) 1-(2,6-Dichlor-4-trifluormethylphenyl)-5-ethoxy-3-methyl-4-methylsulfenyl-pyrazol vom Schmelzpunkt 85°C.

Herstellung der Vorprodukte

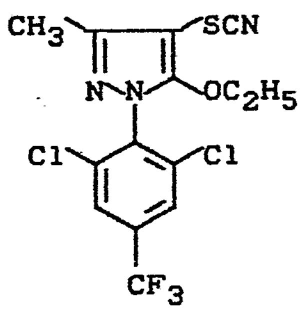

(V-1)

Zu 10,8g (0,034 Mol) 1-(2,6-Dichlor-4-trifluormethyl-phenyl)-5-ethoxy-3-methyl-pyrazol und 5,18g (0,068 Mol) Ammoniumthiocyanat in 150 ml Eisessig tropft man bei +10°C unter Rühren 5,4g (0,034 Mol) Brom in 100 ml Eisessig zu. Nach beendeter Zugabe rührt man 1 Stunde bei +10°C nach. Zur Aufarbeitung gießt man den Ansatz auf Eis, stellt durch Zugabe von konzentrieter wässriger Ammoniaklösung auf pH 9 ein, extrahiert mit Methylenchlorid, trocknet über Natriumsulfat und engt im Vakuum ein. Der ölige Rückstand kristallisiert beim Stehen aus.

Man erhält 13,6g (100% der Theorie) 1-(2,6-Dichlor-4-trifluormethyl-phenyl)-5-ethoxy-3-methyl-4-thiocyano-pyrazol vom Schmelzpunkt 81-83°C.

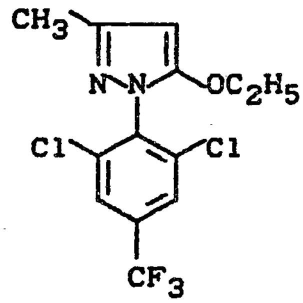

(XVI-1)

Zu einer Lösung von 20g (0,064 Mol) 1-(2,6-Dichlor-4-trifluormethyl-phenyl)-3-methyl-4H-pyrazolin-5-on und 3,9g (0,07 Mol) Kaliumhydroxid in 100 ml Ethanol werden bei Raumtemperatur 9,9g (0,064 Mol) Diethylsulfat getropft. Man läßt das Reaktionsgemisch bis zum vollständigen Umsatz unter Rückfluß rühren. Danach gibt man auf Wasser, extrahiert mit Methylenchlorid, trocknet und engt ein. Nach säulenchromatografischer Trennung mit Methylenchlorid als Laufmittel erhält man 13,6g (62,1% der Theorie) 1-(2,6-Dichlor-4-trifluormethyl-phenyl)-5-ethoxy-3-methyl-pyrazol vom Brechungsindex $n_D^{23}$ = 1,5166.

Beispiel 3

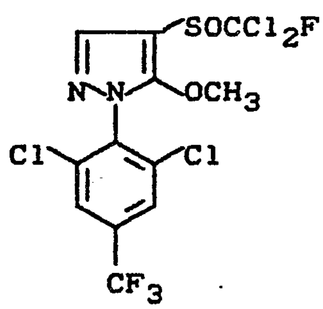

(I-3)

(Verfahren e)

5,0g (0,024 Mol) 4-Dichlorfluormethansulfenyl-1-(2,6-dichlor-4-trifluormethyl-phenyl)-5-methoxy-pyrazol in 50 ml Methylenchlorid werden mit 4,2g (0,024 Mol) m-Chlorperbenzoesäure in 30 ml Methylenchlorid versetzt und über eine Säule mit Methylenchlorid als Laufmittel gereinigt. Die Methylenchloridphase wird eingeengt und der ölige Rückstand kristallisiert nach Zugabe von Petrolether aus. Man erhält 1,5g (27% der Theorie) 4-Dichlorfluormethan-sulfinyl-1-(2,6-dichlor-4-trifluormethylphenyl)-5-methoxy-pyrazol vom Schmelzpunkt 103-106°C.

Beispiel 4

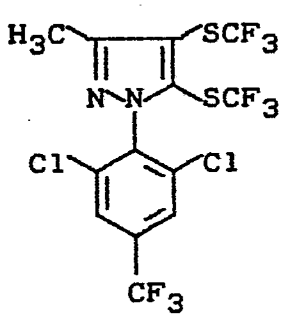

(I-4)

(Verfahren d)

4,75g (10 mMol) 5-Brom-1-(2,6-dichlor-4-trifluormethyl-phenyl)-3-methyl-4-trifluormethylsulfenyl-pyrazol werden in 40 ml absolutem Ether gelöst und bei -78°C unter einer Stickstoffatmosphäre mit 7 ml (11 mMol) 15%-igem n-Butyllithium in Hexan versetzt. Man rührt 1 1/2 Stunden bei -78°C, gibt dann 1,4g (11 mMol) Trifluormethylsulfenylchlorid zur Reaktionsmischung, wobei die Temperatur auf

-60° C ansteigt. Man läßt 2 Stunden bei -78° C nachrühren und erwärmt dann langsam auf Raumtemperatur. Zur Aufarbeitung wird die Reaktionsmischung mit 40 ml Wasser verdünnt und mit Ether extrahiert. Die organische Phase wird mit Wasser und gesättigter Natriumhydrogencarbonatlösung gewaschen, über Magnesiumsulfat getrocknet und im Vakuum eingeengt. Der Rückstand wird säulenchromatographisch auf Kieselgel (Laufmittel= Petrolether/Essigester 20:1) gereinigt.

Man erhält 2,6g (53% der Theorie) 1-(2,6-Dichlor-4-trifluormethylphenyl)-3-methyl-4,5-bis-( trifluormethylsulfenyl)-pyrazol vom Schmelzpunkt 69-71° C.

Herstellung der Vorprodukte

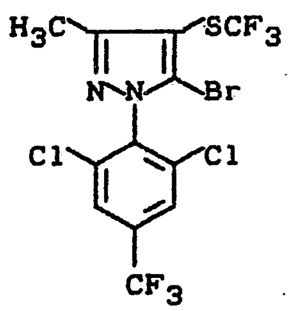

(VII-1)

65,6g (0,16 Mol) 5-Amino-1-(2,6-dichlor-4-trifluormethylphenyl)-3-methyl-4-trifluormethylmercapto-pyrazol werden in 300 ml Tribrommethan gelöst und bei Raumtemperatur mit 54,4 ml (0,458 Mol) tert.-Butylnitrit versetzt. Die Temperatur steigt dabei auf 40-50° C an. Die Reaktionsmischung wird 1 Stunde bei 80° C nachgerührt. Man entfernt das Lösungsmittel im Hochvakuum. Der Rückstand kristallisiert nach dem Verreiben mit Petrolether.

Man erhält 52g (68% der Theorie) an 5-Brom-1-(2,6-dichlor-4-trifluormethylphenyl)-3-methyl-4-trifluormethylmercapto-pyrazol vom Schmelzpunkt 68-70° C.

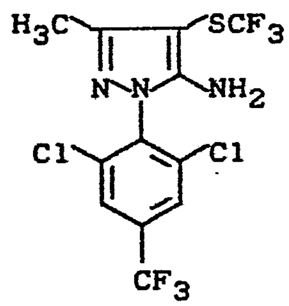

(XVIII)

Zu 62g (0,2 Mol) 5-Amino-1-(2,6-dichlor-4-trifluormethyl-phenyl)-3-methyl-pyrazol in 300g Eisessig werden bei 10-15° C 29g (0,21 Mol) Trifluormethansulfenylchlorid eingeleitet. Nach beendeter Zugabe rührt man weitere 30 Minuten bei Raumtemperatur und gibt dann die Reaktionsmischung auf Wasser. Der Niederschlag wird abgesaugt und getrocknet. Man erhält 71,6g (87% der Theorie) 5-Amino-1-(2,6-dichlor-4-trifluormethylphenyl)-3-methyl-4-trifluormethylsulfenyl-pyrazol vom Schmelzpunkt 148° C.

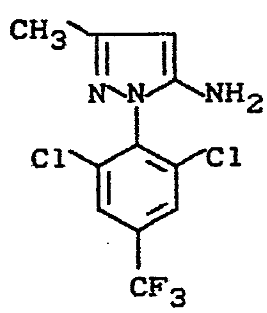

(XX-1)

61,25g (0,25 Mol) 2,6-Dichlor-4-trifluormethylphenylhydrazin und 21g (0,25 Mol) Diacetonitril in 500ml Ethanol werden 20 Stunden unter Rückfluß erhitzt. Zu der erkalteten Reaktionsmischung gibt man 4 ml konzentrierte Schwefelsäure und erhitzt für weitere 4 Stunden auf 60° C. Zur Aufarbeitung wird die Mischung im Vakuum eingedampft, der Rückstand in Chloroform aufgenommen und mit 25%-iger wässriger Ammoniaklösung alkalisch gestellt. Die organische Phase wird abgetrennt und die wässrige Phase mit Chloroform extrahiert. Die vereinigten organischen Phasen werden über Magnesiumsulfat getrocknet und im Vakuum vom Lösungsmittel befreit.

Man erhält 62g (80% der Theorie) an 5-Amino-1-(2,6-dichlor-4-trifluormethylphenyl)-3-methyl-pyrazol als glasartige Substanz.

$^1$H-NMR ($CDCl_3$/TMS) δ= 2,23; 3,50; 5,49; 7,68 ppm.

Entsprechend den Herstellungsbeispielen und gemäß den allgemeinen Angaben zu den Verfahren (a) bis (e) erhält man die folgenden Endprodukte der allgemeinen Formel (I)

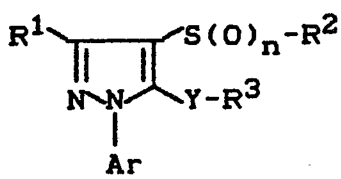

(I)

| Bsp. Nr. | $R^1$ | $-S(O)_n-R^2$ | $-Y-R^3$ | Ar | Fp(°C) |
|---|---|---|---|---|---|
| I-5 | H | $-S-CCl_2F$ | $-O-CH_3$ | 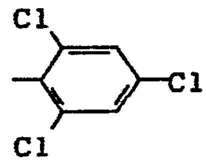 | 104-08 |
| I-6 | H | $-S-CCl_2F$ | $-O-C_2H_5$ | 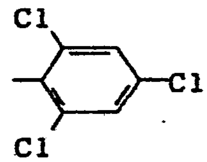 | zähfl.Öl |
| I-7 | $CH_3$ | $-S-CCl_2F$ | $-O-C_2H_5$ | 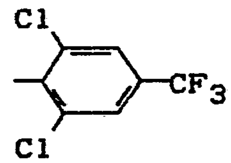 | 46 |
| I-8 | H | $-S-CCl_2F$ | $-O-CH_3$ | 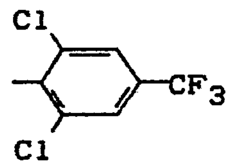 | 92 |
| I-9 | $CH_3$ | $-S-CCl_2F$ | $-O-CH_3$ | 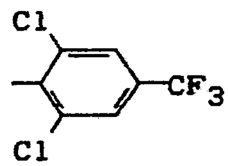 | zähfl.Öl |
| I-10 | H | $-S-CCl_2F$ | $-O-C_2H_5$ | 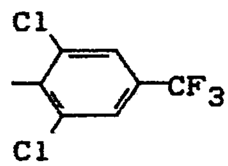 | zähfl.Öl |
| I-11 | H | $-S-CCl_2F$ | $-O-C_3H_7$ | 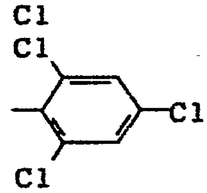 | zähfl.Öl |
| I-12 | $CH_3$ | $-S-CCl_2F$ | $-O-C_3H_7$ | 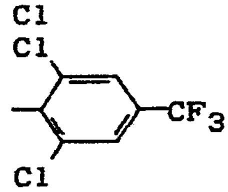 | 73 |
| I-13 | $CH_3$ | $-S-CF_3$ | $-O-CH_3$ | 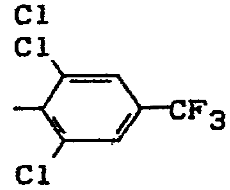 | 78-80 |

| Bsp. Nr. | $R^1$ | $-S(O)_n-R^2$ | $-Y-R^3$ | Ar | Fp(°C) |
|---|---|---|---|---|---|
| I-14 | H | $-S-CClF_2$ | $-O-C_3H_7-i$ | 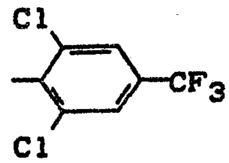 | zähfl.Öl |
| I-15 | H | $-S-CClF_2$ | $-O-CH_3$ | 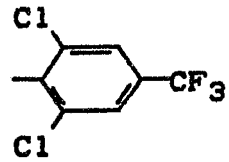 | 88-90 |
| I-16 | $CH_3$ | $-S-CH_3$ | $-O-CH_3$ | 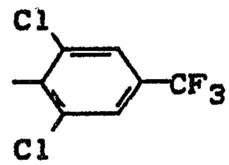 | 68 |
| I-17 | $CH_3$ | $-S-CH_3$ | $-O-C_3H_7-i$ | 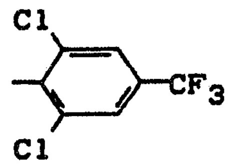 | 48 |
| I-18 | H | $-S-CF_3$ | $-O-C_2H_5$ | 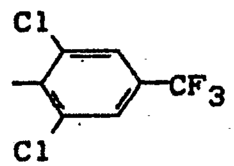 | 60-63 |
| I-19 | H | $-SO-CF_3$ | $-O-C_2H_5$ | 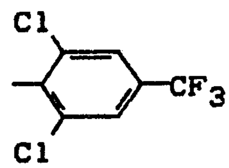 | 73-78 |

Entsprechend den Herstellungsbeispielen und gemäß den allgemeinen Angaben zur Herstellung erhält man die folgenden Vorprodukte der allgemeinen Formel (II)

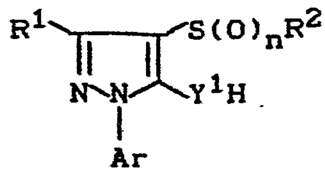

(II)

| Bsp. Nr. | $R^1$ | $-S(O)_n-R^2$ | $-Y^1H$ | Ar | Fp(°C) |
|---|---|---|---|---|---|
| II-2 | H | $-S-CCl_2F$ | -OH | 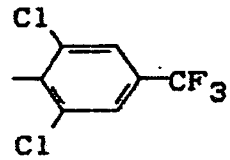 | 165 |
| II-3 | H | $-S-CCl_2F$ | -OH | 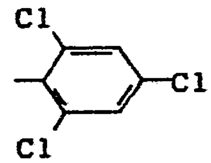 | 140-43 |
| II-4 | H | $-S-CClF_2$ | -OH | 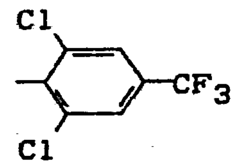 | 125-30 |

Entsprechend den Herstellungsbeispielen und gemäß den allgemeinen Angaben zur Herstellung erhält man die folgenden Vorprodukte der allgemeinen Formel (II a)

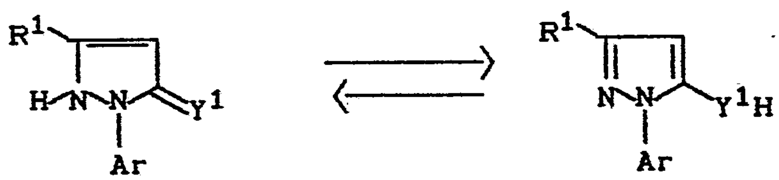

(II a)

| Bsp. Nr. | $R^1$ | $Y^1$ | Ar | Fp(°C) |
|---|---|---|---|---|
| IIa-2 | H | O | 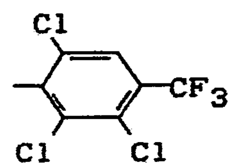 | 183-85 |

Entsprechend den Herstellungsbeispielen und gemäß den allgemeinen Angaben zur Herstellung erhält man die folgenden Vorprodukte der allgemeinen Formel (V)

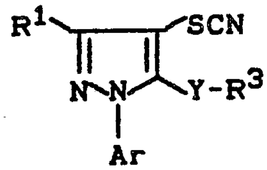 (V)

| Bsp. Nr. | $R^1$ | $-YR^3$ | Ar | Fp(°C) |
|---|---|---|---|---|
| V-2 | $CH_3$ | $-O-CH_3$ | 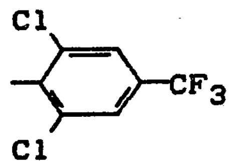 | 95-98 |

Anwendungsbeispiele

In den folgenden Anwendungsbeispielen wurden die nachstehend aufgeführten Verbindungen als Vergleichssubstanzen eingesetzt:

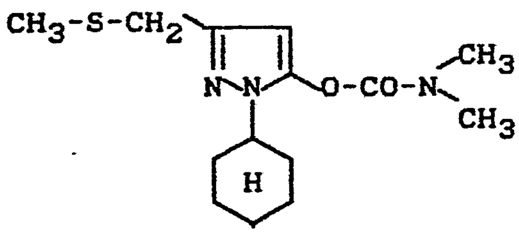

(A)

1-Cyclohexyl-5-[N,N-(dimethyl)-carbamoyloxy]-3-methylthiomethyl-pyrazol (bekannt aus DE-OS 28 39 270);

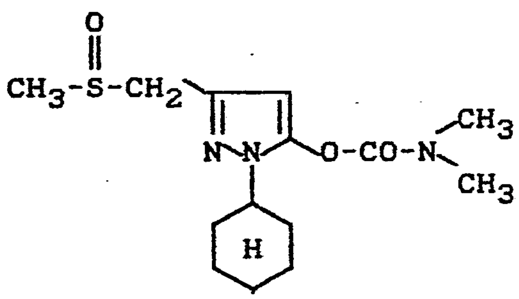

(B)

1-Cyclohexyl-5-[N,N-(dimethyl)-carbamoyloxy]-3-methylsulfinylmethyl-pyrazol (bekannt aus DE-OS 28 39 270).

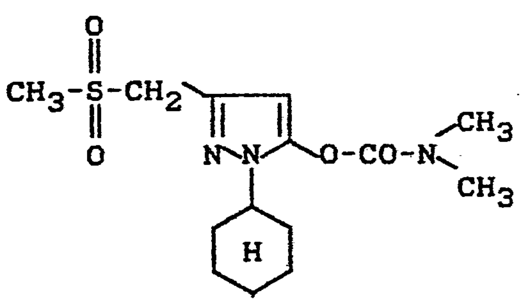

(C)

1-Cyclohexyl-5-[N,N-(dimethyl)-carbamoyloxy]-3-methylsulfonylmethyl-pyrazol (bekannt aus DE-OS 28 39 270).

Beispiel A

$LT_{100}$-Test für Dipteren

Testtiere: Musca domestica (resistent)
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: I-1, I-8, I-10, I-13 und I-16.

Beispiel B

$LD_{100}$-Test

Testtiere: Sitophilus granarius
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem test zeigen z.B. die folgenden Verbindungen der Herstellungsbeispiele überlegene Wirkung gegenüber dem Stand der Technik: I-1, I-6, I-7, I-8, I-9, I-10, I-13, I-14, I-16 und I-17.

0249033

Beispiel C

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: I-1, I-2, I-6, I-7, I-8, I-9, I-10, I-13, I-14, I-15 und I-16.

Beispiel D

Grenzkonzentrations-Test / Bodeninsekten

Testinsekt: Phorbia antiqua-Maden(im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: I-14 und I-15.

Patentansprüche

1. 5-Oxy(thio)-pyrazol-Derivate der allgemeinen Formel (I)

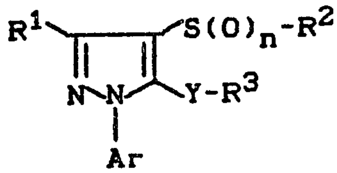

(I)

in welcher

$R^1$ für Wasserstoff, Alkyl oder Halogenalkyl steht,

$R^2$ für gegebenenfalls substituiertes Alkyl, gegebenenfalls substituiertes Alkenyl, gegebenenfalls substituiertes Alkinyl, gegebenenfalls substituiertes Cycloalkyl,gegebenenfalls substituiertes Cycloalkylalkyl, gegebenenfalls substituiertes Aralkyl oder für gegebenenfalls substituiertes Aryl steht,

$R^3$ für gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls substituiertes Aralkyl oder für gegebenenfalls substituiertes Aryl steht,

Ar für jeweils gegebenenfalls substituiertes Phenyl oder Pyridyl steht,

Y für O, S, SO oder $SO_2$ steht und

n für eine Zahl 0, 1 oder 2 steht.

2. 5-Oxy(thio)-pyrazol-Derivate der allgemeinen Formel (I) gemäß Anspruch 1, in welcher

$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^2$ für jeweils gegebenenfalls substituiertes, geradkettiges oder verzweigtes Alkyl, Alkenyl und Alkinyl mit jeweils bis zu 6 Kohlenstoffatomen steht, wobei als Substituenten jeweils in Frage kommen: Halogen; Alkoxy, Alkylthio, Alkylsulfinyl und Alkylsulfonyl mit jeweils 1 bis 4 Kohlenstoffatomen, Cyano und Alkoxycarbonyl mit 1 bis 4 Kohlenstoffatomen im Alkylteil; ferner für jeweils gegebenenfalls einfach oder mehrfach, gleich oder verschieden substituiertes Cycloalkyl und Cycloalkylalkyl mit 3 bis 7 Kohlenstoffatomen im Cycloalkylteil und 1 bis 2 Kohlenstoffatomen im Alkylteil steht, wobei als Substituenten im Cycloalkylteil jeweils in Frage kommen: Halogen, Alkyl, Alkoxy und Alkylthio mit jeweils 1 bis 4 Kohlenstoffatomen; oder für jeweils gegebenenfalls einfach oder mehrfach, gleich oder verschieden substituiertes Phenyl und Phenylalkyl mit 1 bis 4 Kohlenstoffatomen im geradkettigen oder verzweigten

Alkylteil steht, wobei als Phenylsubstituenten jeweils in Frage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

$R^3$ für jeweils gegebenenfalls substituiertes, geradkettiges oder verzweigtes Alkyl, Alkenyl und Alkinyl mit jewils bis zu 6 Kohlenstoffatomen steht, wobei als Substituenten jeweils in Frage kommen: Halogen; Alkoxy, Alkylthio, Alkylsulfinyl und Alkylsulfonyl mit jeweils 1 bis 4 Kohlenstoffatomen, Cyano und Alkoxycarbonyl mit 1 bis 4 Kohlenstoffatomen im Alkylteil; ferner für jeweils gegebenenfalls einfach oder mehrfach, gleich oder verschieden substituiertes Cycloalkyl und Cycloalkylalkyl mit 3 bis 7 Kohlenstoffatomen im Cycloalkylteil und 1 bis 2 Kohlenstoffatomen im Alkylteil steht, wobei als Substituenten im Cycloalkylteil jeweils genannt seien: Halogen, Alkyl, Alkoxy und Alkylthio mit jeweils 1 bis 4 Kohlenstoffatomen; oder für jeweils gegebenenfalls einfach oder mehrfach, gleich oder verschieden substituiertes Phenyl und Phenylalkyl mit 1 bis 4 Kohlenstoffatomen im geradkettigen oder verzweigten Alkylteil steht, wobei als Phenylsubstituenten

jeweils in Frage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_mR^4$

wobei

$R^4$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht;

Y für O, S, SO oder $SO_2$ steht und

n für eine Zahl 0, 1 oder 2 steht.

3) 5-Oxy(thio)pyrazol-Derivate der allgemeinen Formel (I) gemäß Anspruch 1, in welcher

$R^1$ für Wasserstoff, Methyl, Ethyl, n- oder i-Propyl und Trifluormethyl steht;

$R^2$ für Methyl, Ethyl; n- oder i-Propyl; n-, i-, s- oder t-Butyl; n- oder i-Pentyl; n- oder i-Hexyl, Allyl, Propenyl, Butenyl, Propargyl oder Butinyl steht, die jeweils einfach oder mehrfach, gleich oder verschieden substituiert sein können durch Fluor, Chlor, Methoxy, Ethoxy, Methylthio, Methylsulfinyl, Methylsulfonyl, Cyano, Methoxycarbonyl oder Ethoxycarbonyl; ferner für Cyclopropyl, Cyclopentyl, Cyclohexyl, Cyclopropylmethyl, Cyclopentylmethyl oder Cyclohexylmethyl steht, die jeweils einfach oder zweifach, gleich oder verschieden durch Fluor, Chlor oder Methyl substituiert sein können; oder für jeweils gegebenenfalls ein- bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl,

Methoxy, Methylthio, Trifluormethyl, Methylsulfinyl, Methylsulfonyl, Trifluormethoxy, Trifluormethylthio, Trifluormethylsulfinyl oder Trifluormethylsulfonyl;

$R^3$ für Methyl, Ethyl; n- oder i-Propyl; n-, i-, s- oder t-Butyl; n- oder i-Pentyl; n- oder i-Hexyl, Allyl, Propenyl, Butenyl, Propargyl oder Butinyl steht, die jeweils einfach oder mehrfach, gleich oder verschieden substituiert sein können durch Fluor, Chlor, Methoxy, Ethoxy, Methylthio, Methylsulfinyl, Methylsulfonyl, Cyano, Methoxycarbonyl oder Ethoxycarbonyl;
ferner für Cyclopropyl, Cyclopentyl, Cyclohexyl, Cyclopropylmethyl, Cyclopentylmethyl oder Cyclohexylmethyl steht, die jeweils einfach oder zweifach, gleich oder verschieden durch Fluor, Chlor oder Methyl substituiert sein können;
oder für jeweils gegebenenfalls ein- bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl;

Ar für jeweils gegebenenfalls ein- bis fünffach, gleich oder verschieden substituiertes Phenyl, 2-Pyridyl, 3-Pyridyl oder 4-Pyridyl steht, wobei als Substituenten in Frage kommen:

0249033

Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_mR^4$,

wobei

$R^4$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und

m für eine Zahl 0, 1 oder 2 steht;

Y für O, S, SO oder $SO_2$ steht und

n für eine Zahl 0, 1 oder 2 steht.

4. Verfahren zur Herstellung von 5-Oxy(thio)-pyrazol-Derivaten der allgemeinen Formel (I)

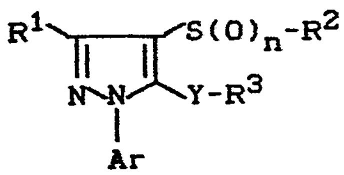

(I)

in welcher

$R^1$ für Wasserstoff, Alkyl oder Halogenalkyl steht,

$R^2$ für gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls substituiertes Aralkyl oder für gegebenenfalls substituiertes Aryl steht,

$R^3$ für gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl,gegebenenfalls substituiertes Aralkyl oder für gegebenenfalls substituiertes Aryl steht,

Ar für jeweils gegebenenfalls substituiertes Phenyl oder Pyridyl steht,

Y für O, S, SO oder $SO_2$ steht und

n für eine Zahl 0, 1 oder 2 steht,

dadurch gekennzeichnet, daß man

a) zum Erhalt der 5-Oxy(thio)-pyrazol-Derivate der Formel (Ia)

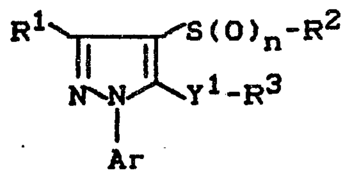

(Ia)

in welcher

$R^1$, $R^2$ ,$R^3$ ,Ar und der Index n die oben angegebene Bedeutung haben und

$Y^1$ für Sauerstoff oder Schwefel steht,

Pyrazolin-one(thione) der tautomeren Formeln (II)

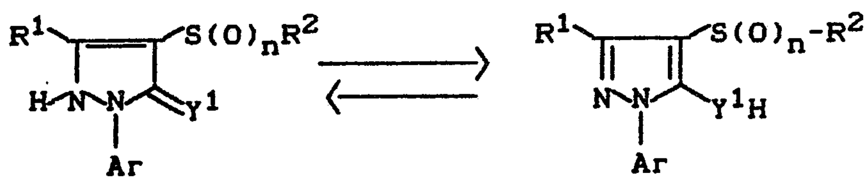

(II)

in welcher

$R^1$, $R^2$, Ar, $Y^1$ und der Index n die oben angegebene Bedeutung haben,

mit Alkylierungsmitteln der Formel (III)

$$A - R^3 \qquad (III)$$

in welcher

$R^3$ die oben angegebene Bedeutung hat und

A für eine elektronenziehende Abgangsgruppe steht,

in Gegenwart eines Verdünnungsmittels und gegebenenfalls in Gegenwart eines Säurebindemittels umsetzt, wobei je nach Reaktionsbedingungen auch die entsprechenden zu (Ia) tautomeren N-alkylierten-Derivate der Formel (IV)

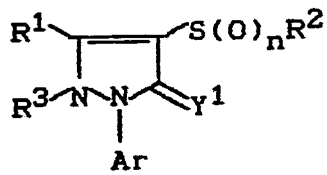

(IV)

in welcher

$R^1$, $R^2$, $R^3$, Ar, $Y^1$ und der Index n die oben angegebene Bedeutung haben

erhalten werden können,

b) oder daß man zum Erhalt der 5-Oxy(thio)-pyrazol-Derivate der Formel (Ib)

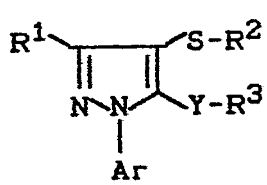

(Ib)

in welcher

$R^1$, $R^2$, $R^3$, Y und Ar die oben angegebene Bedeutung haben,

(α) Pyrazol-Derivate der Formel (V)

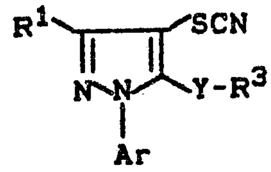

(V)

in welcher

$R^1$, $R^3$, Y und Ar die oben angegebene Bedeutung haben,

oder

(β) Bis-pyrazol-Derivate der Formel (VI)

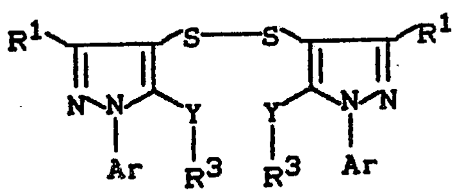

(VI)

$R^1$, $R^3$, Y und Ar die oben angegebene Bedeutung haben,

mit Alkylierungsmitteln der Formel (III a)

$$A - R^2 \qquad \text{(III a)}$$

in welcher

$R^2$ und A die oben angegebene Bedeutung haben,

gegebenenfalls in Gegenwart eines Verdünnungsmittels sowie in Gegenwart eines geeigneten Reduktionsmittels und in Gegenwart einer Base umsetzt,

(c) oder daß man zum Erhalt der 5-Oxy(thio)-pyrazol-Derivate der Formel (I) Halogen-pyrazol-Derivate der Formel (VII)

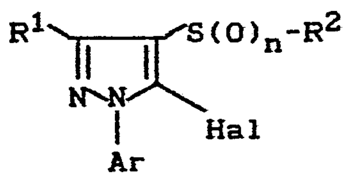

(VII)

in welcher

$R^1$, $R^2$, Ar und der Index n die oben angegebene Bedeutung hat und

Hal für Fluor, Chlor oder Brom steht,

mit Verbindungen der Formel (VIII)

$$H - Y - R^3 \qquad (VIII)$$

in welcher

$R^3$ und Y die oben angegebene Bedeutung haben,

in Gegenwart eines Verdünnungsmittels und in Gegenwart eines Säurebindemittels umsetzt,

d) oder daß man zum Erhalt der 5-Thio-pyrazol-Derivate der Formel (Ic)

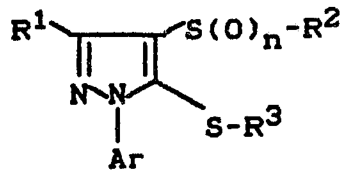

(I c)

in welcher

$R^1$, $R^2$, $R^3$, Ar und der Index n die oben angegebene Bedeutung haben,

Halogen-pyrazol-Derivate der Formel (VII)

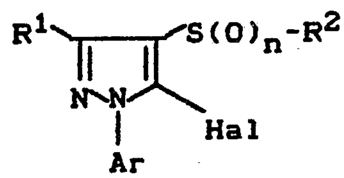

(VII)

in welcher

$R^1$, $R^2$, Ar und der Index n die oben angegebene Bedeutung haben und

Hal für Fluor, Chlor oder Brom steht,

mit Sulfenylhalogeniden der Formel (IX)

$$Hal^1 - S - R^3 \qquad (IX)$$

in welcher

$R^3$ die oben angegebene Bedeutung hat und

$Hal^1$ für Chlor oder Brom steht,

in Gegenwart einer Lithium-organischen Verbindungen und in Gegenwart eines Verdünnungsmittels umsetzt,

(e) oder daß man zum Erhalt der am Schwefel in 4- oder/und 5-Stellung oxidierten 5-Oxy(thio)-pyrazol-Derivate der Formel (I), Mercapto-Derivate der Formel (I d)

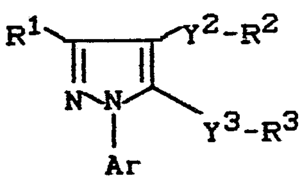

(I d)

worin

$R^1$, $R^2$, $R^3$ und Ar die oben angegebene Bedeutung haben und

$Y^2$ für Schwefel und

$Y^3$ für die Bedeutung von Y steht,

oder

$Y^2$ für die Gruppierungen SO oder $SO_2$ steht und

$Y^3$ für Schwefel steht,

nach bekannten Methoden in üblicher Weise oxidiert.

0249033

5. Schädlingsbekämpfungsmittel, gekennzeichnet durch einen Gehalt an mindestens einem 5-Oxy(thio)-pyrazol der Formel (I).

6. Insektizide, akarizide und nematizide Mittel, gekennzeichnet durch einen Gehalt an mindestens einem 5-Oxy(thio)-pyrazol der Formel (I).

7. Verfahren zur Bekämpfung von Insekten und/oder Spinnentieren und/oder Nematoden, dadurch gekennzeichnet, daß man 5-Oxy(thio)-pyrazole der Formel (I) auf Insekten und/oder Spinnentiere und/oder Nematoden und/oder deren Lebensraum einwirken läßt.

8. Verwendung von 5-Oxy(thio)-pyrazolen der Formel (I) zur Bekämpfung von Insekten und/oder Spinnentieren und/oder Nematoden.

9. Verfahren zur Herstellung von Schädlingsbekämpfungsmitteln, dadurch gekennzeichnet, daß man 5-Oxy(thio)-pyrazole der Formel (I) mit Streckmitteln und/oder oberflächenaktiven Mitteln vermischt.

Europäisches Patentamt

# EUROPÄISCHER RECHERCHENBERICHT

Nummer der Anmeldung
0249033
EP 87 10 6682

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | CHEMICAL ABSTRACTS, Band 88, Nr. 5, 30. Januar 1978, Seite 465, Spalte 1, Zusammenfassungsnr. 37373s, Columbus, Ohio, US; D. LADUREE et al.: "Condensation reactions with carbonions of sulfonyl ketones. Chemical properties of the addition compounds", in Zusammenhang mit CHEMICAL SUBSTANCE INDEX, Pp-Z, Band 88, Januar-Juni 1978, Seite 4361C5, Spalte 1 "3-ethyl-5 (methylthio)-1-phenyl-4-(phenylsulfonyl)-1H-pyrazole", & RECUEIL DES TRAVAUX CHIMIQUES DES PAYS BAS 1977, 96(10), 254-258 --- | 1-3 | C 07 D 231/28<br>C 07 D 401/04<br>A 01 N 43/56 //<br>C 07 D 231/22<br>C 07 C 109/04<br>C 07 D 231/18<br>C 07 D 231/44<br>C 07 D 231/38 |
| X | PATENT ABSTRACTS OF JAPAN, Band 6, Nr. 90 (C-104)[968], 27. Mai 1982; & JP - A - 57 21373 (SANKYO K.K.) 04.02.1982 --- | 4 | RECHERCHIERTE SACHGEBIETE (Int. Cl.4) |
| X | CHEMICAL ABSTRACTS, Band 75, Nr. 5, 2. August 1971, Seite 498, Zusammenfassungsnr. 35870j, Columbus, Ohio, US; P. GIORI et al.: "Synthesis and antifungal properties of pyrazolyl mono- and disulfides II", & FARMACO, ED. SCI. 1971, 26(4), 276-293 --- -/- | 4 | C 07 D 231/00<br>A 01 N 43/00<br>C 07 D 401/00 |

Der vorliegende Recherchenbericht wurde für alle Patentansprüche erstellt.

| Recherchenort | Abschlußdatum der Recherche | Prüfer |
|---|---|---|
| BERLIN | 26-08-1987 | VAN AMSTERDAM L.J.P. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung
0249033
EP 87 10 6682

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 72, Nr. 15, 13. April 1970, Seite 393, Spalte 1, Zusammenfassungsnr. 78937s, Columbus, Ohio, US; I.T. KAY et al.: "Synthesis of potential insecticides. II. Carbamic esters of 4-alkythiopyrazolones", & J. CHEM. SOC. 1970,(3),445-448<br>--- | 1,5-9 | |
| A | EP-A-0 154 115 (BAYER AG)<br>* Anspruch 5, & DE - A - 3 402 308 (Kat. D) *<br>--- | 1 | |
| A,D | EP-A-0 009 634 (BAYER AG)<br>& DE - A - 2 839 270 (Kat. D)<br>----- | | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| BERLIN | 26-08-1987 | VAN AMSTERDAM L.J.P. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument